(19)

Europäisches
Patentamt
European
Patent Office
Office européen
des brevets

(11)    EP 3 403 577 A1

(12)    **EUROPEAN PATENT APPLICATION**
published in accordance with Art. 153(4) EPC

(43) Date of publication:
　21.11.2018  Bulletin 2018/47

(51) Int Cl.:
　*A61B 5/055* (2006.01)　　*A61B 5/00* (2006.01)
　*G01R 33/32* (2006.01)

(21) Application number: 16886614.3

(22) Date of filing: 27.09.2016

(86) International application number:
　PCT/KR2016/010827

(87) International publication number:
　WO 2017/126771 (27.07.2017 Gazette 2017/30)

(84) Designated Contracting States:
　**AL AT BE BG CH CY CZ DE DK EE ES FI FR GB
　GR HR HU IE IS IT LI LT LU LV MC MK MT NL NO
　PL PT RO RS SE SI SK SM TR**
　Designated Extension States:
　**BA ME**
　Designated Validation States:
　**MA MD**

(30) Priority:  21.01.2016  KR 20160007535

(71) Applicant: **Samsung Electronics Co., Ltd.
　Suwon-si, Gyeonggi-do 16677 (KR)**

(72) Inventor: **HWANG, Jin-young
　Suwon-si
　Gyeonggi-do 16663 (KR)**

(74) Representative: **D'Halleweyn, Nele Veerle Trees
　Gertrudis et al
　Arnold & Siedsma
　Bezuidenhoutseweg 57
　2594 AC The Hague (NL)**

(54)  **MAGNETIC RESONANCE IMAGING APPARATUS AND METHOD FOR SHIMMING OF
　　　MAGNETIC RESONANCE IMAGING APPARATUS**

(57)　　Provided is a shimming method performed by a magnetic resonance imaging (MRI) apparatus, the shimming method including applying a magnetic field and a high frequency to an object through a bore of the MRI apparatus including at least one magnet and an RF coil and obtaining a static magnetic field map corresponding to the magnetic field formed in the bore; performing shimming using a plurality of shim channels based on the static magnetic field map; receiving a free induction decay (FID) signal from the RF coil; and determining a state of the magnetic field based on the FID signal and controlling whether to stop shimming based on the determined state.

FIG. 5

```
              ┌─────────┐
              │  START  │
              └─────────┘
                   │
   ┌───────────────────────────────────┐
   │  ACQUIRE STATIC MAGNETIC FIELD MAP │── S510
   └───────────────────────────────────┘
                   │
   ┌───────────────────────────────────┐
   │ PERFORM SHIMMING USING PLURALITY OF│── S520
   │           SHIM CHANNELS            │
   └───────────────────────────────────┘
                   │
   ┌───────────────────────────────────┐
   │         RECEIVE FID SIGNAL         │── S530
   └───────────────────────────────────┘
                   │
   ┌───────────────────────────────────┐
   │  DETERMINE STATE OF MAGNETIC FIELD │
   │  BASED ON FID SIGNAL AND CONTROL   │── S540
   │    WHETHER TO STOP SHIMMING        │
   └───────────────────────────────────┘
                   │
              ┌─────────┐
              │   END   │
              └─────────┘
```

EP 3 403 577 A1

**Description**

TECHNICAL FIELD

The present disclosure relates to a magnetic resonance imaging apparatus and a shimming method of the magnetic resonance imaging apparatus.

More specifically, the present disclosure relates to a magnetic resonance imaging apparatus capable of effective homogenization of a magnetic field in a bore in the magnetic resonance imaging apparatus and a shimming method of the magnetic resonance imaging apparatus.

BACKGROUND ART

A magnetic resonance imaging (MRI) apparatus captures an image of a target object by using a magnetic field. Since the MRI apparatus is capable of creating three-dimensional images of bones, discs, joints, ligaments, or the like at a user-desired angle, the MRI apparatus is widely used to make a correct disease diagnosis.

The MRI apparatus obtains a magnetic resonance (MR) signal, reconstructs the obtained MR signal into an image, and outputs the image. In more detail, the MRI apparatus obtains the MR signal by using a high-frequency multi-coil including radio frequency (RF) coils, permanent-magnets, superconducting magnets, gradient coils, etc.

Specifically, a high frequency signal generated by applying a pulse sequence for generating a radio frequency signal to a high frequency multi coil is applied to an object, and a MR image is reconstructed by sampling a magnetic resonance signal (a MR signal) generated in response to the applied high-frequency signal.

One important factor in determining the quality of the MR image is the field homogeneity of the magnetic field formed in the bore of the MRI apparatus. That is, a static magnetic field formed in the bore needs to be homogeneous at each position such that a non-distorted magnetic resonance image may be obtained. When the static magnetic field is distorted, a resonance frequency value at each coordinate of the bore varies, which makes it impossible to apply an accurate resonance frequency. At this time, correcting non-homogeneity of the static magnetic field is referred to as shimming, and the MRI apparatus may separately include a shim coil for shimming.

The shim coil may include a passive shim coil and a higher-order shim coil. When a MRI system is first installed, the magnetic field of the bore is non-homogenously distributed. At this time, to homogenize the inhomogeneous magnetic field, the passive shim coil is used to fill a magnet with small pieces of iron plate so that the magnetic field becomes evenly distributed. After completing passive shimming by using the passive shim coil, when a human body or other matter is placed in the magnetic field, the magnetic field is distorted again. To homogenize the distorted magnetic field again, the higher-order shim coil may be used to form and compensate for an additional magnetic field.

The higher-order shim coil has a plurality of channels. A shimming method using the high-order shim coils is used to adjust coefficients of the plurality of channels such that a static magnetic field map has the same frequency at each position. Coefficients of shim channels may be adjusted by controlling the magnitude of a current flowing in each shim coil.

When shimming using the shim coil is performed, a shimming operation ends without performing a separate feedback process after repeating the above-described shimming process at least once. Subsequently, magnetic resonance imaging is performed according to a predetermined protocol. Hereinafter, the protocol for performing magnetic resonance imaging with respect to an object will be referred to as an "imaging protocol". When shimming is performed once and then magnetic resonance imaging is performed according to the imaging protocol, a good image quality may not be guaranteed because magnetic resonance imaging is obtained without checking whether the magnetic field is homogeneous or non-homogenous. On the other hand, when shimming is performed at least once and then magnetic resonance imaging is performed according to the imaging protocol, although a magnetic field having a good homogeneity may be obtained when shimming is performed only once, and thus, shimming needs to be repeated at least once unconditionally, which may cause waste of time.

That is, since there is no standard for determining the number of times of repeatedly performing shimming, when shimming is performed a fixed number of times, a magnetic field with the best homogeneity may not always be formed, and waste of time may be caused by unnecessarily repeating shimming.

Therefore, there is a need to provide a method and apparatus for effectively performing shimming.

DESCRIPTION OF EMBODIMENTS

TECHNICAL PROBLEM

Provided are a magnetic resonance imaging (MRI) apparatus capable of improving homogeneity of a magnetic field while reducing the number of times of performing shimming and a shimming method of the magnetic resonance imaging apparatus.

SOLUTION TO PROBLEM

According to an aspect of the present disclosure, a shimming method performed by a magnetic resonance imaging (MRI) apparatus includes applying a magnetic field and a high frequency to an object through a bore of the MRI apparatus including at least one magnet and an RF coil and obtaining a static magnetic field map corresponding to the magnetic field formed in the bore; performing shimming using a plurality of shim channels based on the static magnetic field map; receiving a free induction decay (FID) signal from the RF coil; and determining a state of the magnetic field based on the FID signal and controlling whether to stop shimming based on the determined state.

BRIEF DESCRIPTION OF DRAWINGS

FIG. 1 is a block diagram of a general magnetic resonance imaging (MRI) system.
FIG. 2 is a block diagram of a communication unit according to an embodiment.
FIG. 3 is a block diagram showing a MRI apparatus according to an embodiment.
FIG. 4 is another block diagram showing a MRI apparatus according to an embodiment.
FIG. 5 is a flowchart illustrating a repetitive high-order shimming method of a MRI apparatus according to an embodiment.
FIG. 6A is a diagram schematically illustrating an inhomogeneous magnetic field formed in a bore before a MRI apparatus completes shimming, according to an embodiment.
FIG. 6B is a diagram showing respective shim channel functions for a MRI apparatus to shim the inhomogeneous magnetic field of FIG. 6A, according to an embodiment.
FIG. 6C is a diagram schematically showing a magnetic field formed in a homogenized bore after a MRI apparatus completed shimming, according to an embodiment.
FIG. 7 is a diagram showing a method performed by a MRI apparatus of performing shimming using a shim coil, according to an embodiment.
FIG. 8 is a flowchart illustrating a method performed by a MRI apparatus of controlling repetitive high-order shimming, according to an embodiment.
FIG. 9A is a graph schematically showing a free induction decay (FID) signal obtained by a MRI apparatus in an inhomogeneous state of a static magnetic field before completing shimming, according to an embodiment.
FIG. 9B is a graph schematically showing a FID signal obtained by a MRI apparatus after homogenously completing shimming of a static magnetic field, according to an embodiment.
FIG. 10 is a graph showing a frequency difference between a FID signal for water and a FID signal for fat obtained by a MRI apparatus, according to an embodiment.
FIG. 11 is a flowchart illustrating a method performed by a MRI apparatus of controlling repetitive high-order shimming according to whether each of a FID signal waveform for water and a FID signal waveform for fat matches a reference FID signal waveform, according to an embodiment.
FIG. 12 is a flowchart illustrating a method performed by a MRI apparatus of receiving a shimming control reference from a user and controlling repetitive high-order shimming, according to an embodiment.

BEST MODE

According to an aspect of the present disclosure, a shimming method performed by a magnetic resonance imaging (MRI) apparatus includes applying a magnetic field and a high frequency to an object through a bore of the MRI apparatus including at least one magnet and an RF coil and obtaining a static magnetic field map corresponding to the magnetic field formed in the bore; performing shimming using a plurality of shim channels based on the static magnetic field map; receiving a free induction decay (FID) signal from the RF coil; and determining a state of the magnetic field based on the FID signal and controlling whether to stop shimming based on the determined state.
The performing of the shimming may include: calculating offsets of the plurality of shim channels based on the static magnetic field map; and applying a current to the plurality of shim channels based on the calculated offsets.
The controlling whether to stop shimming may include controlling whether to stop shimming by using a FID signal for each of fat and water received from the RF coil.
The FID signal for each of fat and water may be an FID signal with respect to a specific part of the object.
The controlling whether to stop shimming may include controlling to stop shimming when a difference between a frequency value of the FID signal for fat and a frequency value of the FID signal for water matches a reference value.

The controlling whether to stop shimming may include controlling to stop shimming when a waveform of the FID signal for each of fat and water matches a waveform of a reference FID signal.

The controlling whether to stop shimming may include controlling to stop shimming when an FID signal other than the FID signal for each of fat and water is not detected.

The controlling whether to stop shimming may include receiving a reference for controlling a user to stop shimming.

According to another aspect of the present disclosure, a magnetic resonance imaging (MRI) apparatus includes a bore including at least one magnet and an RF coil and configured to apply a magnetic field and a high frequency to an object; and a control unit configured to obtain a static magnetic field map corresponding to the magnetic field formed in the bore based on a high frequency signal received from the RF coil, to perform shimming using a plurality of shim channels based on the static magnetic field map, to determine a state of the magnetic field based on the FID signal, and to control whether to stop shimming based on the determined state.

The control unit may be configured to calculate offsets of the plurality of shim channels based on the static magnetic field map and apply a current to the plurality of shim channels based on the calculated offsets.

The control unit may be configured to control whether to stop shimming by using a FID signal for each of fat and water received from the RF coil.

The FID signal for each of fat and water may be an FID signal with respect to a specific part of the object.

The control unit may be configured to control to stop shimming when a difference between a frequency value of the FID signal for fat and a frequency value of the FID signal of water matches a reference value.

The control unit may be configured to control to stop shimming when a waveform of the FID signal for each of fat and water matches a waveform of a reference FID signal.

The control unit may be configured to control to stop shimming when an FID signal other than the FID signal for each of fat and water is not detected.

The control unit may be configured to receive a reference for controlling a user to stop shimming.

MODE OF DISCLOSURE

Advantages and features of one or more embodiments of the disclosure and methods of accomplishing the same may be understood more readily by reference to the following detailed description of the embodiments and the accompanying drawings. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the disclosure will only be defined by the appended claims.

Terms used herein will now be briefly described and then one or more embodiments of the disclosure will be described in detail.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the disclosure. Thus, the terms used herein have to be defined based on the meaning of the terms together with the description throughout the specification.

When a part "includes" or "comprises" an element, unless there is a particular description contrary thereto, the part may further include other elements, not excluding the other elements. Also, the term "unit" in the embodiments of the disclosure means a software component or hardware component such as a field-programmable gate array (FPGA) or an application-specific integrated circuit (ASIC), and performs a specific function. However, the term "unit" is not limited to software or hardware. The "unit" may be formed to be in an addressable storage medium, or may be formed to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

Reference will now be made in detail to embodiments, examples of which are illustrated in the accompanying drawings. In this regard, the present embodiments may have different forms and should not be construed as being limited to the descriptions set forth herein. In the following description, well-known functions or constructions are not described in detail so as not to obscure the embodiments with unnecessary detail.

In the present specification, an "image" may refer to multi-dimensional data composed of discrete image elements (e.g., pixels in a two-dimensional (2D) image and voxels in a three-dimensional (3D) image). For example, the image may be a medical image of an object captured by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, and a technician who repairs a medical apparatus.

Furthermore, in the present specification, an "MR image" refers to an image of an object obtained by using the nuclear magnetic resonance principle.

Furthermore, in the present specification, a "pulse sequence" refers to continuity of signals repeatedly applied by an MRI apparatus. The pulse sequence may include a time parameter of a radio frequency (RF) pulse, for example, repetition time (TR) or echo time (TE).

Furthermore, in the present specification, a "pulse sequence schematic diagram" shows an order of events that occur in an MRI apparatus. For example, the pulse sequence schematic diagram may be a diagram showing an RF pulse, a gradient magnetic field, an MR signal, or the like according to time.

The term "static magnetic field" as used herein means a magnetic field formed in a bore by a main magnet such as permanent magnets, resistive electromagnets, and superconducting electromagnets, etc. In a MRI apparatus, the term "static magnetic field" may be referred to as a "main magnetic field". Also, the static magnetic field may be referred to as a static magnetic field.

Also, the term "static magnetic field map" as used herein refers to a map indicating a region in which a static magnetic field is formed.

Further, "shimming" as used herein refers to adjusting homogeneity of a magnetic field formed in a bore. An MRI apparatus may include a shim coil to adjust the homogeneity of the magnetic field. The shim coil may include a plurality of shim channels. The MRI apparatus may adjust the homogeneity of the magnetic field by adjusting a magnitude of current flowing through each shim channel.

Specifically, the MRI apparatus may perform shimming by acquiring a static field map and adjusting a magnitude of current flowing through a shim channel to form a magnetic field capable of offsetting a distorted portion of the static magnetic field.

Also, the term "free induction decay (FID) signal" as used herein refers to a transverse relaxation signal of hydrogen atomic nucleus (1H) spins measured in a direction of an x-y plane of a bore. When 1D Fourier transform is performed on the transverse relaxation signal, a mixed signal of materials included in the x-y plane may be obtained.

An MRI system is an apparatus for acquiring a sectional image of a part of an object by expressing, in a contrast comparison, a strength of a MR signal with respect to a radio frequency (RF) signal generated in a magnetic field having a specific strength. For example, when an RF signal that only resonates a specific atomic nucleus (for example, a hydrogen atomic nucleus) is emitted for an instant toward the object placed in a strong magnetic field and then such emission stops, an MR signal is emitted from the specific atomic nucleus, and thus the MRI system may receive the MR signal and acquire an MR image. The MR signal denotes an RF signal emitted from the object. An intensity of the MR signal may be determined according to a density of a predetermined atom (for example, hydrogen) of the object, a relaxation time T1, a relaxation time T2, and a flow of blood or the like.

MRI systems include characteristics different from those of other imaging apparatuses. Unlike imaging apparatuses such as CT apparatuses that acquire images according to a direction of detection hardware, MRI systems may acquire 2D images or 3D volume images that are oriented toward an optional point. MRI systems do not expose objects or examiners to radiation, unlike CT apparatuses, X-ray apparatuses, position emission tomography (PET) apparatuses, and single photon emission CT (SPECT) apparatuses, may acquire images having high soft tissue contrast, and may acquire neurological images, intravascular images, musculoskeletal images, and oncologic images that are required to precisely capturing abnormal tissues.

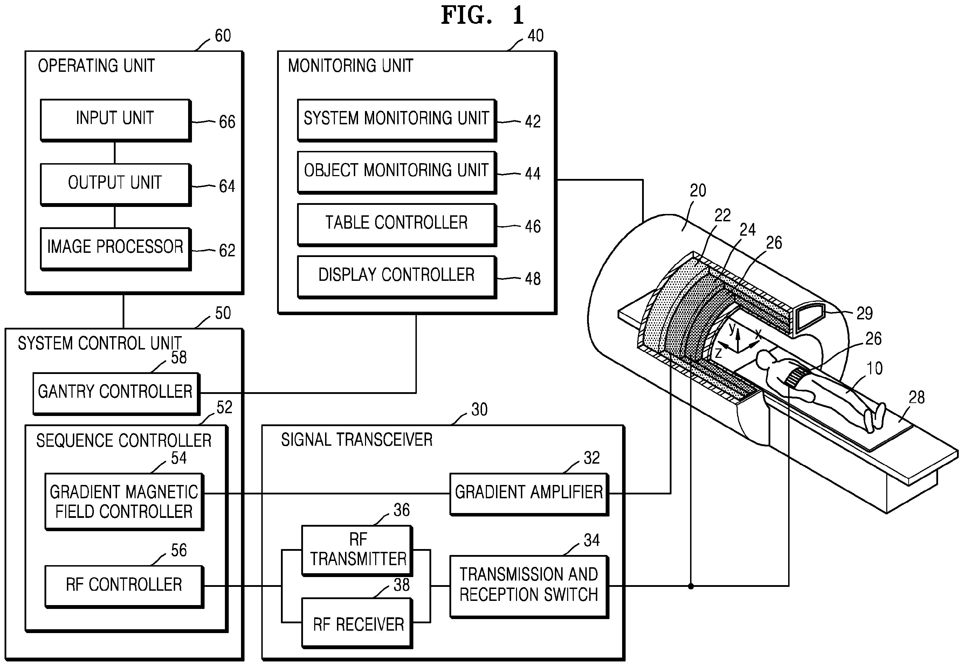

FIG. 1 is a block diagram of a general MRI system. Referring to FIG. 1, the general MRI system may include a gantry 20, a signal transceiver 30, a monitoring unit 40, a system control unit 50, and an operating unit 60.

The gantry 20 prevents external emission of electromagnetic waves generated by a main magnet 22, a gradient coil 24, and an RF coil 26. A magnetostatic field and a gradient magnetic field are formed in a bore in the gantry 20, and an RF signal is emitted toward an object 10.

The main magnet 22, the gradient coil 24, and the RF coil 26 may be arranged in a predetermined direction of the gantry 20. The predetermined direction may be a coaxial cylinder direction. The object 10 may be disposed on a table 28 that is capable of being inserted into a cylinder along a horizontal axis of the cylinder.

The main magnet 22 generates a magnetostatic field or a static magnetic field for aligning magnetic dipole moments of atomic nuclei of the object 10 in a constant direction. A precise and accurate MR image of the object 10 may be obtained due to a magnetic field generated by the main magnet 22 being strong and uniform.

The gradient coil 24 includes X, Y, and Z coils for generating gradient magnetic fields in X-, Y-, and Z-axis directions crossing each other at right angles. The gradient coil 24 may provide location information of each region of the object 10 by differently inducing resonance frequencies according to the regions of the object 10. Also, the gradient coil 24 may include a shim coil. The shim coil (not shown) may be attached inside the gradient coil 24. In an embodiment, the shim coil may include a linear channel of X, Y, Z and a shim channel of $Z^2$, ZX, ZY, $X^2$-$Y^2$, XY corresponding to the higher order coil. An MRI apparatus may adjust homogeneity of the magnetic field by using the shim coil.

The RF coil 26 may emit an RF signal toward a patient and receive an MR signal emitted from the patient. In detail, the RF coil 26 may transmit, toward atomic nuclei included in the patient and having precessional motion, an RF signal having the same frequency as that of the precessional motion, stop transmitting the RF signal, and then receive an MR signal emitted from the atomic nuclei included in the patient.

For example, to transit an atomic nucleus from a low energy state to a high energy state, the RF coil 26 may generate and apply an electromagnetic wave signal having an RF corresponding to a type of the atomic nucleus, for example, an RF signal, to the object 10. When the electromagnetic wave signal generated by the RF coil 26 is applied to the atomic nucleus, the atomic nucleus may transit from the low energy state to the high energy state. Then, when electromagnetic waves generated by the RF coil 26 disappear, the atomic nucleus to which the electromagnetic waves were applied transits from the high energy state to the low energy state, thereby emitting electromagnetic waves having a Lamor frequency. In other words, when the applying of the electromagnetic wave signal to the atomic nucleus is stopped, an energy level of the atomic nucleus is changed from a high energy level to a low energy level, and thus the atomic nucleus may emit electromagnetic waves having a Lamor frequency. The RF coil 26 may receive electromagnetic wave signals from atomic nuclei included in the object 10.

The RF coil 26 may be realized as one RF transmitting and receiving coil having both a function of generating electromagnetic waves having an RF corresponding to a type of an atomic nucleus and a function of receiving electromagnetic waves emitted from an atomic nucleus. Alternatively, the RF coil 26 may be realized as a transmission RF coil having a function of generating electromagnetic waves having an RF corresponding to a type of an atomic nucleus, and a reception RF coil having a function of receiving electromagnetic waves emitted from an atomic nucleus.

The RF coil 26 may be fixed to the gantry 20 or may be detachable. When the RF coil 26 is detachable, the RF coil 26 may be an RF coil for a part of the object, such as a head RF coil, a chest RF coil, a leg RF coil, a neck RF coil, a shoulder RF coil, a wrist RF coil, or an ankle RF coil.

The RF coil 26 may communicate with an external apparatus via wires and/or wirelessly, and may also perform dual tune communication according to a communication frequency band.

The RF coil 26 may be a birdcage coil, a surface coil, or a transverse electromagnetic (TEM) coil according to structures.

The RF coil 26 may be a transmission exclusive coil, a reception exclusive coil, or a transmission and reception coil according to methods of transmitting and receiving an RF signal.

The RF coil 26 may be an RF coil having various numbers of channels, such as 16 channels, 32 channels, 72 channels, and 144 channels.

The gantry 20 may further include a display 29 disposed outside the gantry 20 and a display (not shown) disposed inside the gantry 20. The gantry 20 may provide predetermined information to the user or the object 10 through the display 29 and the display respectively disposed outside and inside the gantry 20.

The signal transceiver 30 may control the gradient magnetic field formed inside the gantry 20, i.e., in the bore, according to a predetermined MR sequence, and control transmission and reception of an RF signal and an MR signal.

The signal transceiver 30 may include a gradient amplifier 32, a transmission and reception switch 34, an RF transmitter 36, and an RF receiver 38.

The gradient amplifier 32 drives the gradient coil 24 included in the gantry 20, and may supply a pulse signal for generating a gradient magnetic field to the gradient coil 24 under the control of a gradient magnetic field controller 54. By controlling the pulse signal supplied from the gradient amplifier 32 to the gradient coil 24, gradient magnetic fields in X-, Y-, and Z-axis directions may be synthesized.

The RF transmitter 36 and the RF receiver 38 may drive the RF coil 26. The RF transmitter 36 may supply an RF pulse in a Lamor frequency to the RF coil 26, and the RF receiver 38 may receive an MR signal received by the RF coil 26.

The transmission and reception switch 34 may adjust transmitting and receiving directions of the RF signal and the MR signal. For example, the transmission and reception switch 34 may emit the RF signal toward the object 10 through the RF coil 26 during a transmission mode, and receive the MR signal from the object 10 through the RF coil 26 during a reception mode. The transmission and reception switch 34 may be controlled by a control signal output by an RF controller 56.

The monitoring unit 40 may monitor or control the gantry 20 or devices mounted on the gantry 20. The monitoring unit 40 may include a system monitoring unit 42, an object monitoring unit 44, a table controller 46, and a display controller 48.

The system monitoring unit 42 may monitor and control a state of the magnetostatic field, a state of the gradient magnetic field, a state of the RF signal, a state of the RF coil 26, a state of the table 28, a state of a device measuring body information of the object 10, a power supply state, a state of a thermal exchanger, and a state of a compressor.

The object monitoring unit 44 monitors a state of the object 10. In detail, the object monitoring unit 44 may include a camera for observing a movement or position of the object 10, a respiration measurer for measuring the respiration of the object 10, an electrocardiogram (ECG) measurer for measuring the electrical activity of the object 10, or a temperature measurer for measuring a temperature of the object 10.

The table controller 46 controls a movement of the table 28 where the object 10 is positioned. The table controller 46 may control the movement of the table 28 according to a sequence control of a sequence controller 52. For example, during moving imaging of the object 10, the table controller 46 may continuously or discontinuously move the table 28 according to the sequence control of the sequence controller 52, and thus the object 10 may be photographed in a field of view (FOV) larger than that of the gantry 20.

The display controller 48 controls the display 29 disposed outside the gantry 20 and the display disposed inside the gantry 20. In detail, the display controller 48 may control the display 29 and the display to be on or off, and may control a screen image to be output on the display 29 and the display. Also, when a speaker is located inside or outside the gantry 20, the display controller 48 may control the speaker to be on or off, or may control sound to be output via the speaker.

The system control unit 50 may include the sequence controller 52 for controlling a sequence of signals formed in the gantry 20, and a gantry controller 58 for controlling the gantry 20 and the devices mounted on the gantry 20.

The sequence controller 52 may include the gradient magnetic field controller 54 for controlling the gradient amplifier 32, and the RF controller 56 for controlling the RF transmitter 36, the RF receiver 38, and the transmission and reception switch 34. The sequence controller 52 may control the gradient amplifier 32, the RF transmitter 36, the RF receiver 38, and the transmission and reception switch 34 according to a pulse sequence received from the operating unit 60. Here, the pulse sequence includes all information required to control the gradient amplifier 32, the RF transmitter 36, the RF receiver 38, and the transmission and reception switch 34. For example, the pulse sequence may include information for strength, an application time, and application timing of a pulse signal applied to the gradient coil 24.

The operating unit 60 may request the system control unit 50 to transmit pulse sequence information while controlling an overall operation of the MRI system.

The operating unit 60 may include an image processor 62 for receiving and processing the MR signal received by the RF receiver 38, an output unit 64, and an input unit 66.

The image processor 62 may process the MR signal received from the RF receiver 38 to generate MR image data of the object 10.

The image processor 62 receives the MR signal received by the RF receiver 38 and performs any one of various signal processes, such as amplification, frequency transformation, phase detection, low frequency amplification, and filtering, on the received MR signal.

The image processor 62 may arrange digital data in a k space (for example, also referred to as a Fourier space or a frequency space) of a memory, and rearrange the digital data into image data via 2D or 3D Fourier transformation.

The image processor 62 may perform a composition process or a difference calculation process on the image data if required. The composition process may be an addition process performed on a pixel or a maximum intensity projection (MIP) process performed on a pixel. The image processor 62 may store not only the rearranged image data but also image data on which a composition process or a difference calculation process is performed, in a memory (not shown) or an external server.

The image processor 62 may perform any of the signal processes on the MR signal in parallel. For example, the image processor 62 may perform a signal process on a plurality of MR signals received by a multi-channel RF coil in parallel to rearrange the plurality of MR signals into image data.

The output unit 64 may output image data generated or rearranged by the image processor 62 to the user. The output unit 64 may also output information required for the user to manipulate the MRI system, such as a user interface (UI), user information, or object information. The output unit 64 may be a speaker, a printer, a cathode-ray tube (CRT) display, a liquid crystal display (LCD), a plasma display panel (PDP), an organic light-emitting device (OLED) display, a field emission display (FED), a light-emitting diode (LED) display, a vacuum fluorescent display (VFD), a digital light processing (DLP) display, a flat panel display (FPD), a 3-dimensional (3D) display, a transparent display, or any one of other various output devices that are well known to one of ordinary skill in the art.

The user may input object information, parameter information, a scan condition, a pulse sequence, or information about image composition or difference calculation by using the input unit 66. The input unit 66 may be a keyboard, a mouse, a track ball, a voice recognizer, a gesture recognizer, a touch screen, or any one of other various input devices that are well known to one of ordinary skill in the art.

The signal transceiver 30, the monitoring unit 40, the system control unit 50, and the operating unit 60 are separate components in FIG. 1, but it will be obvious to one of ordinary skill in the art that respective functions of the signal transceiver 30, the monitoring unit 40, the system control unit 50, and the operating unit 60 may be performed by another component. For example, the image processor 62 converts the MR signal received from the RF receiver 38 into a digital signal in FIG. 1, but alternatively, the conversion of the MR signal into the digital signal may be performed by the RF receiver 38 or the RF coil 26.

The gantry 20, the RF coil 26, the signal transceiver 30, the monitoring unit 40, the system control unit 50, and the operating unit 60 may be connected to each other by wire or wirelessly, and when they are connected wirelessly, the MRI system may further include an apparatus (not shown) for synchronizing clock signals therebetween. Communication between the gantry 20, the RF coil 26, the signal transceiver 30, the monitoring unit 40, the system control unit 50, and the operating unit 60 may be performed by using a high-speed digital interface, such as low voltage differential signaling (LVDS), asynchronous serial communication, such as a universal asynchronous receiver transmitter (UART), a low-delay network protocol, such as error synchronous serial communication or a controller area network (CAN), optical communication, or any of other various communication methods that are well known to one of ordinary skill in the art.

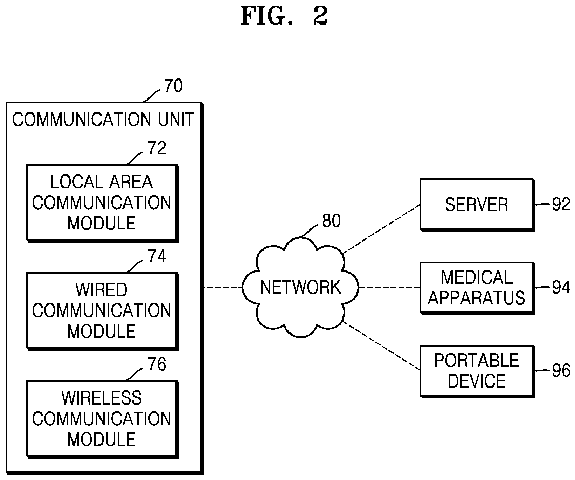

FIG. 2 is a block diagram of a communication unit 70 according to an embodiment. Referring to FIG. 2, the communication unit 70 may be connected to at least one selected from the gantry 20, the signal transceiver 30, the monitoring unit 40, the system control unit 50, and the operating unit 60 of FIG. 1.

The communication unit 70 may transmit and receive data to and from a hospital server or another medical apparatus in a hospital, which is connected through a picture archiving and communication system (PACS), and perform data communication according to the digital imaging and communications in medicine (DICOM) standard.

As shown in FIG. 2, the communication unit 70 may be connected to a network 80 by wire or wirelessly to communicate with a server 92, a medical apparatus 94, or a portable device 96.

In detail, the communication unit 70 may transmit and receive data related to the diagnosis of an object through the network 80, and may also transmit and receive a medical image captured by the medical apparatus 94, such as a CT apparatus, an MRI apparatus, or an X-ray apparatus. In addition, the communication unit 70 may receive a diagnosis history or a treatment schedule of the object from the server 92 and use the same to diagnose the object. The communication unit 70 may perform data communication not only with the server 92 or the medical apparatus 94 in a hospital, but also with the portable device 96, such as a mobile phone, a personal digital assistant (PDA), or a laptop of a doctor or patient.

Also, the communication unit 70 may transmit information about a malfunction of the MRI system or about a medical image quality to a user through the network 80, and receive a feedback regarding the information from the user.

The communication unit 70 may include at least one component enabling communication with an external apparatus. For example, the communication unit 70 may include a local area communication module 72, a wired communication module 74, and a wireless communication module 76.

The local area communication module 72 refers to a module for performing local area communication with an apparatus within a predetermined distance. Examples of local area communication technology according to an embodiment of the disclosure include, but are not limited to, a wireless local area network (LAN), Wi-Fi, Bluetooth, ZigBee, Wi-Fi direct (WFD), ultra wideband (UWB), infrared data association (IrDA), Bluetooth low energy (BLE), and near field communication (NFC).

The wired communication module 74 refers to a module for performing communication by using an electric signal or an optical signal. Examples of wired communication technology according to an embodiment of the disclosure include wired communication techniques using a twisted pair cable, a coaxial cable, and an optical fiber cable, and other well known wired communication techniques.

The wireless communication module 76 transmits and receives a wireless signal to and from at least one selected from a base station, an external apparatus, and a server in a mobile communication network. Here, the wireless signal may be a voice call signal, a video call signal, or data in any one of various formats according to transmission and reception of a text/multimedia message.

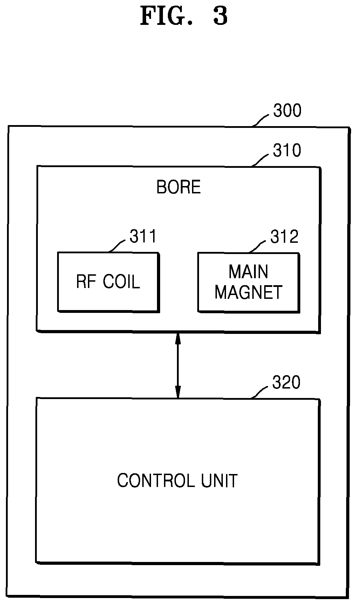

FIG. 3 is a block diagram showing a MRI apparatus 300 according to an embodiment.

The MRI apparatus 300 according to an embodiment may include a bore 310 and a control unit 320. The MRI apparatus 300 may be any medical imaging apparatus capable of controlling shimming to perform imaging of a magnetic resonance image. Specifically, the MRI apparatus 300 may correspond to the MRI system shown in FIG. 1.

The bore 310 may include at least one magnet and an RF coil to apply a magnetic field and a high frequency to an object. Specifically, the bore 310 may be a cylindrical tube included in the gantry 20, and may include an RF coil 311 and a main magnet 312. Also, the bore 310 may include a gradient coil (24 in FIG. 1) and a shim coil (not shown) in addition to the RF coil 311 and the main magnet 312.In this regard, the RF coil 311 and the main magnet 312 may respectively correspond to the main magnet 22 and the RF coil 26 of FIG. 1.

The control unit 320 may obtain a main magnetic field map corresponding to the magnetic field formed in the bore 310 based on a high frequency signal received from the RF coil 311, perform shimming using a plurality of shim channels (not shown) based on the main magnetic field map, determine a state of the magnetic field based on a free induction decay (FID) signal received from the RF coil 311, and control whether to stop shimming based on determination. Also, the control unit 320 may correspond to the system control unit 50 of FIG. 1. Specifically, the control unit 320 may correspond to the gantry controller 58. Also, the control unit 320 may generally control an operation of the MRI apparatus 300.

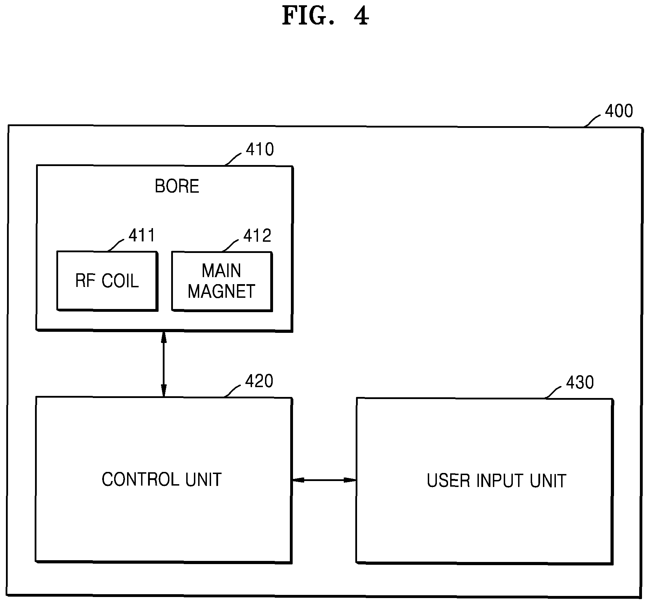

FIG. 4 is another block diagram showing a MRI apparatus 400 according to an embodiment. The MRI apparatus 400 may further include a user input unit 430 in comparison with the MRI apparatus 300 shown in FIG. 3. A bore 410 and a control unit 420 of the MRI apparatus 400 may respectively correspond to the bore 310 and the control unit 320 of the MRI apparatus 300 and will not be described in detail.

The user input unit 430 may receive a stop control standard for repetitive high-order shimming from a user. The user input unit 430 may display a user input window for receiving the stop control standard for repetitive high-order shimming from the user. The user input unit 430 may be included in the operating unit 60. Specifically, the user input unit 430 may be included in the input unit 66. The control unit 420 may control whether to stop shimming by using the control reference of the repetitive high-order shimming input through the user input unit 430.

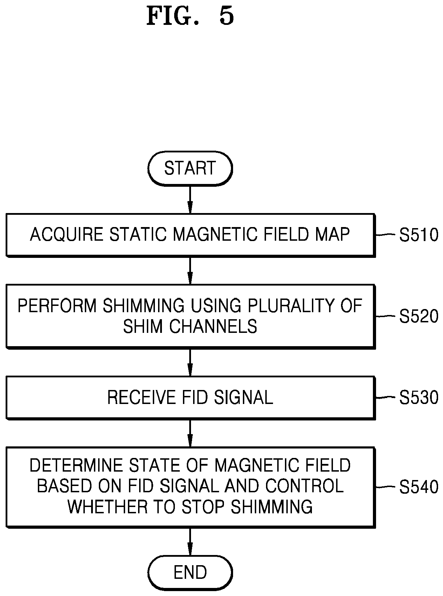

Hereinafter, detailed operations of the MRI apparatuses 300 and 400 according to embodiments will be described in detail with reference to FIGS. 5 to 12.

FIG. 5 is a flowchart illustrating a repetitive high-order shimming method used by a MRI apparatus according to an embodiment.

Referring to FIG. 5, the MRI apparatus 400 may acquire a static magnetic field map formed in the bore 410 (S510). The MRI apparatus 400 may form a static magnetic field in the bore 410 using a main magnet 412. The MRI apparatus 400 may acquire the static magnetic field map formed in the bore 410 using the RF coil 311.

The MRI apparatus 400 may perform shimming using a plurality of shim channels of a shim coil (S520). Specifically, a shimming method using the shim coil may include calculating a coefficient of each shim channel based on the static magnetic field map. In this regard, the coefficient of the shim channel may be a set value of the shim channel for forming a magnetic field to be applied to offset an inhomogeneous magnetic field in performing shimming.

A current corresponding to a coefficient value may be applied to each shim channel to form a magnetic field capable of offsetting the inhomogeneous magnetic field. That is, the MRI apparatus 400 may form a homogenous magnetic field by applying a magnetic field in a direction opposite to the inhomogeneous magnetic field through the shim coil.

Only the shimming method of the MRI apparatus 400 of calculating the coefficient of the shim channel based on the static magnetic field map and offsetting the inhomogeneous magnetic field is described. However, the shimming method is not limited thereto, and shimming may be performed in various ways.

The MRI apparatus 400 may receive a FID signal using a RF coil 411 (S530). For example, when an electromagnetic signal generated by the RF coil 411 is applied to a certain atomic nucleus, the nucleus may transit from a low energy state to a high energy state. Thereafter, when electromagnetic waves generated by the RF coil 411 disappears, the atomic nucleus to which the electromagnetic waves were applied may emit an electromagnetic wave having a Larmor frequency while transiting from the high energy state to the low energy state. At this time, a transverse relaxation signal of an atomic nuclear spin measured by the RF coil 411 in a direction of an x-y plane of the bore 410 may be the FID signal. The FID signal may have a unique value for each tissue of an object according to magnitude of a main magnetic field applied to the bore 410.

The MRI apparatus 400 may determine a state of the magnetic field based on the FID signal and control whether to stop shimming (S540). The FID signal may have the unique value for each tissue of the object according to the magnitude of the main magnetic field. When the static magnetic field is in a homogeneous state after shimming, the FID signal detected in a specific tissue of the object may be detected with the same value as a unique FID signal value of the specific tissue. On the other hand, when shimming is not completed and the static magnetic field is inhomogeneous, the FID signal detected in the specific tissue of the object due to a distortion of the static magnetic field may be detected as a value different from the unique FID signal value of the specific tissue.

In an embodiment, the MRI apparatus 400 may control whether to stop repetitive high-order shimming by using a characteristic having a unique FID signal for each specific tissue.

In an embodiment, the MRI apparatus 400 may control whether to stop repetitive high-order shimming based on a FID signal for each of water and fat. Specifically, a shimming stop control operation may be performed by the control unit 420.

The MRI apparatus 400 may acquire an image using a magnetic moment of a $^1$H atomic nucleus. This is because a high density of $^1$H is present in a human body and the $^1$H atomic nucleus emits a very strong magnetic resonance signal. Therefore, the most MRI apparatuses 400 acquire image signals by applying a frequency adjacent to the Larmor frequency of $^1$H at a resonance frequency. Since water and fat include $^1$H in each molecule, water and fat may emit relatively large signals when acquiring magnetic resonance images. Therefore, when the MRI apparatus 400 acquires the FID signal, it is possible to detect two peaks corresponding to the FID signal of each of fat and water.

At this time, since the human body has a relatively large amount of water FID signals and a relatively small amount of fat FID signals, the MRI apparatus 400 may detect two peaks having different amplitudes.

In an embodiment, the MRI apparatus 400 may control to stop repetitive high-order shimming when a frequency difference between the FID signals of water and fat corresponding to the two peaks corresponds to a certain value. Specifically, the shimming stop control operation may be performed by the control unit 420.

When intensity of the main magnetic field is 1 Tesla, a resonance frequency of the hydrogen atomic nucleus may be 42.58 MHz. Therefore, when the frequency difference between fat and the water is 3.4 ppm, the frequency difference (hereinafter $\Delta f$) in the FID signal of each of fat and water under 3 Tesla of the main magnetic field may be obtained as shown in Equation 1.

[Equation 1]

$$\triangle f = 42.58 MHz/Tesla * 3 Tesla * 3.4 * 10^{-6} \doteqdot 434 Hz$$

The MRI apparatus 400 may control to stop repetitive high-order shimming when $\Delta f$ corresponds to a reference value.

In an embodiment, the MRI apparatus 400 may control whether to stop repetitive high-order shimming based on the FID signal of each of fat and water with respect to a 'specific part' of the object. Since the FID signal of fat measured in the human body is small in size, it is possible to observe the two peaks more clearly when measuring the FID signal at the specific part (for example, abdomen, leg, etc.) where a relatively large amount of fat is distributed.

In an embodiment, the MRI apparatus 400 may control to stop repetitive high-order shimming when an acquired FID signal waveform for fat and water matches a reference FID signal waveform. Specifically, the shimming stop control operation may be performed by the control unit 420.

When the FID signal waveform of water is referred to as a first peak and the FID signal waveform for fat is referred to as a second peak, in a case where an interval between the first and second peaks in the FID signal acquired by the MRI apparatus 400 matches an interval between first and second peaks in the reference FID signal waveform, the MRI apparatus 400 may control to stop repetitive high-order shimming. At this time, the 'interval between two peaks' may correspond to the above-described $\Delta f$. When shimming is completed, since the first peak and the second peak have values at the Larmor frequency of water and fat, respectively, the interval between the two peaks may correspond to $\Delta f$.

Also, when heights of remaining peaks except for the first and second peaks in the FID signal acquired by the MRI apparatus 400 respectively match heights of remaining peaks except for the first and second peaks of the reference FID signal waveform, the MRI apparatus 400 may control to stop repetitive high-order shimming. When shimming is completed, since other waveforms other than the FID signal of water and fat are not detected, magnitude of values of the remaining peaks except for the first and second peaks may be small or the remaining peaks may not be detected.

In an embodiment, the MRI apparatus 400 may control to stop repetitive high order shimming when the number of peaks of the FID signal received by the RF coil 411 matches the number of peaks of the reference FID signal.

The fact that the waveform of the FID signal acquired by the MRI apparatus 400 matches the waveform of the reference FID signal indicates that not only exact numerical values matches but also a difference between numerical values matches an error range that may be tolerated by one of ordinary skilled in the art.

In an embodiment, the MRI apparatus 400 may control to stop shimming when no FID signal other than the FID signals of fat and water is detected. Currently, the most MRI apparatus 400 may obtain magnetic resonance signals by applying the frequency adjacent to the Larmor frequency of the $^1$H atomic nucleus. Accordingly, when the MRI apparatus 400 acquires the FID signal under a homogeneous static magnetic field state, only the FID signals of fat and water including $^1$H may be obtained. This is because the MRI apparatus 400 may not detect FID signals of other materials having the Larmor frequency in a frequency band different from that of the $^1$H atomic nucleus.

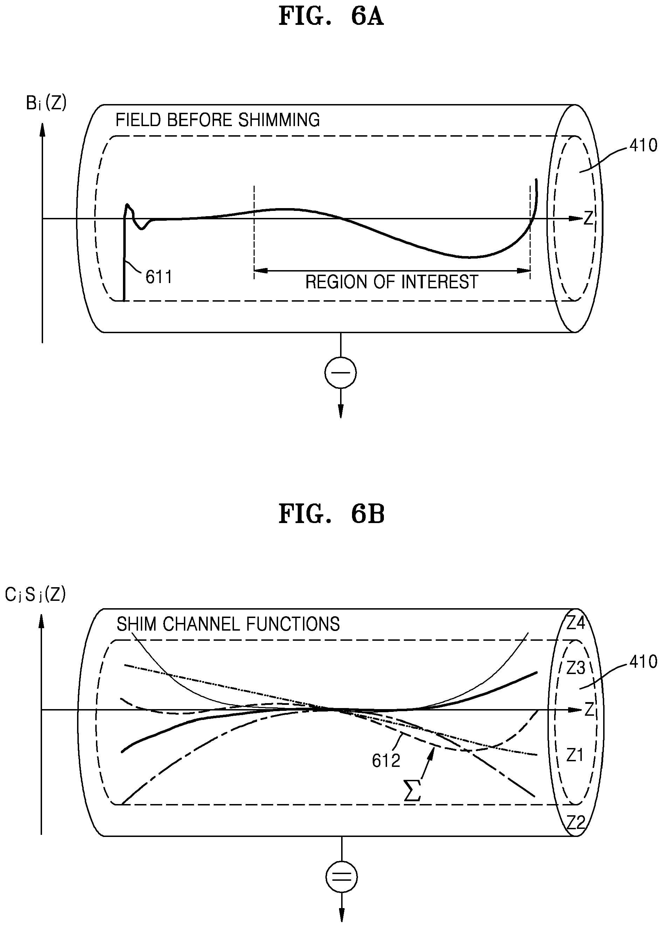

FIG. 6A is a diagram schematically illustrating an inhomogeneous magnetic field of the bore 410 before the MRI apparatus 400 completes shimming, according to an embodiment.

Referring to FIG. 6A, a z-axis direction of a graph may be a direction in which the table 28 on which a patient who is an object of magnetic resonance imaging is located enters the gantry 20 as shown in FIG. 1. A region of interest (ROI) may represent a predetermined region within the gantry 20 where a static magnetic field needs to be homogenized since a user intends to acquire a MR signal. The ROI may include a space in which a body part of the patient to be imaged is located and may be a partial or whole space within the gantry 20. Before completion of shimming, a magnetic field of the bore 410 may not be homogeneous as shown in FIG. 6A. Specifically, a graph 611 shows a magnetic field in a corresponding region of the gantry 20. Referring to the graph 611, the magnetic field B(z) may be inhomogeneous along the region (the z-axis direction) of the gantry 20. Since an image acquired by the MRI apparatus 400 may be distorted in a state where the magnetic field of the ROI is inhomogeneous, good quality may not be expected.

When the field of the bore 410 is inhomogeneous as shown in FIG. 6A, each shim channel may form shim channel functions that may offset the inhomogeneous magnetic field. For example, the shim coil may include a shim channel including a plurality of linear coils and a high-order coil. The MRI apparatus 400 may calculate a coefficient of each shim channel that may offset the inhomogeneous magnetic field. That is, the MRI apparatus 400 may calculate a coefficient value of a shim channel function such that a sum ($\Sigma$) of a magnetic field formed by each shim channel may match the inhomogeneous magnetic field before shimming.

FIG. 6B is a diagram showing respective shim channel functions for the MRI apparatus 400 to shim an inhomogeneous magnetic field of FIG. 6A, according to an embodiment. Referring to FIG. 6B, a plurality of shim channel functions $C_j S_j(z)$ may be formed along a region (a z-axis direction) of the gantry 20. In in $C_j S_j(z)$, $S_j$ may correspond to each of shim channel functions Z1, Z2, Z3, and Z4, and $C_j$ may correspond to a coefficient of each of the shim channel functions Z1, Z2, Z3, and Z4. The bore 410 may include a plurality of shim channels. Each of the shim channels may perform shimming according to each of corresponding shim channel functions Z1, Z2, Z3, and Z4. In this regard, since a degree of homogeneity of a magnetic field is different for each region in the bore 410, the shim channel functions Z1, Z2, Z3, and Z4 may be formed differently for each shim channel. The MRI apparatus 400 may use a plurality of shim channel functions to allow the sum $\Sigma$ of the plurality of shim channel functions to match an inhomogeneous magnetic field before shimming. Referring to a graph 612, the graph 612 is a magnetic field constituting a sum of the plurality of shim channel functions Z1, Z2, Z3, and Z4, and may have a shape corresponding to a shape of the graph 611 in a ROI of FIG. 6A. For example, the graph 612 may have the same or similar shape as the graph 611.

In an embodiment, the MRI apparatus 400 may form shim channel functions in a manner that applies current of magnitude corresponding to each coefficient value to each shim channel.

Although FIG. 6B shows four shim channel functions Z1, Z2, Z3, and Z4, the number of shim channel functions is not limited thereto. The MRI apparatus 400 may include a plurality of shim channel functions.

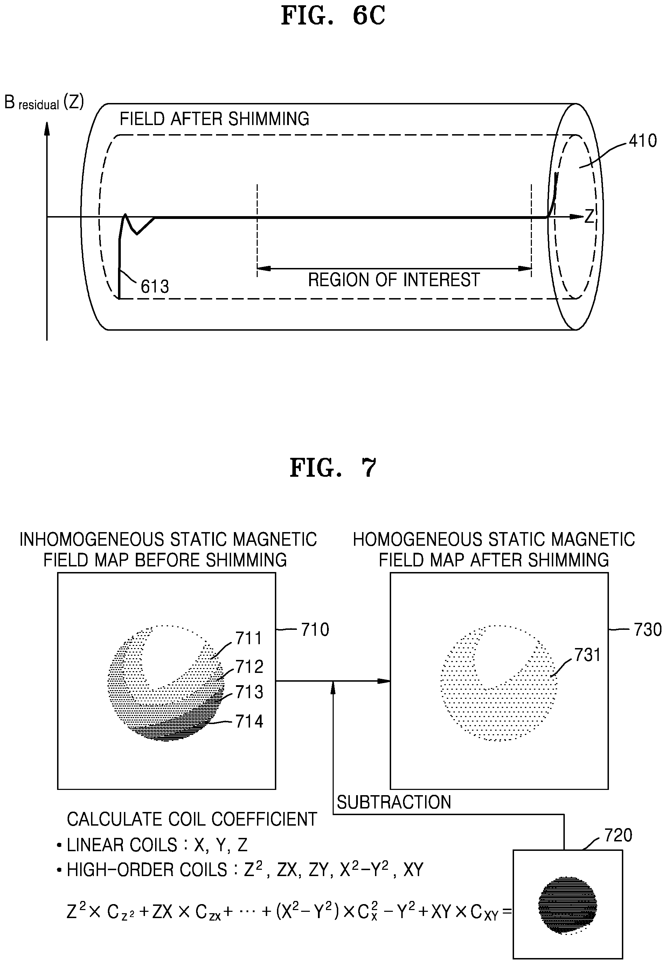

FIG. 6C is a diagram schematically showing a magnetic field $B_{residual}(z)$ of the homogenized bore 410 after the MRI apparatus 400 completed shimming, according to an embodiment.

Specifically, FIG. 6C shows the magnetic field $B_{residual}(z)$ after shimming along a region of the gantry 20. The MRI apparatus 400 may perform shimming in such a manner that the sum $\Sigma$ of shim channel functions shown in FIG. 6B is subtracted from an inhomogeneous magnetic field shown in FIG. 6A. Referring to a graph 613, since the magnetic field $B_{residual}(z)$ after shimming has a homogeneous magnetic field in a ROI, an undistorted image may be obtained.

In an embodiment, the MRI apparatus 400 may perform a process of FIGS. 6A to 6C as a shimming operation of one time and achieve shimming by repeating such a shimming operation.

FIG. 7 is a diagram showing a method performed by the MRI apparatus 400 of performing shimming using a shim coil, according to an embodiment.

FIG. 7 shows a shimming process on a single material phantom.

The MRI apparatus 400 may acquire a static magnetic field map 710 in an x-y plane direction (2D) of a bore before shimming.

Referring to FIG. 7, the static magnetic field map 710 shows a state in which a static magnetic field is inhomogeneous since an offset occurs in the static magnetic field. The higher the frequency offset, the darker the concentration may be displayed. Accordingly, in the static magnetic field map 710, the static magnetic field is distorted in a circular shape, and the higher the frequency offset occurs closer from 711 to 714. The MRI apparatus 400 may perform shimming to form a homogeneous static magnetic field by offsetting the offset. The MRI apparatus 400 may calculate a coefficient of each shim channel function for shimming. The MRI apparatus 400 may include shim coils including linear coils of X, Y, Z and high-order coils of $Z^2$, ZX, ZY, $X^2-Y^2$, and XY. The MRI apparatus 400 may calculate an offset of each shim channel based on the inhomogeneous static magnetic field map 710 before shimming. The MRI apparatus 400 may calculate respective coefficients $C_x$, $C_y$, $C_z$, $C_{z2}$, $C_{zx}$, $C_{zy}$, $C_{x2-y2}$, and $C_{xy}$ of the linear coils of X, Y, Z and the high-order coils of $Z^2$, ZX, ZY, $X^2-Y^2$, and XY such that the shim coils may form a magnetic field capable of offsetting the offset. The MRI apparatus 400 may form the magnetic field by each shim channel by applying current to each coil based on the calculated coefficients $C_x$, $C_y$, $C_z$, $C_{z2}$, $C_{zx}$, $C_{zy}$, $C_{x2-y2}$, and $C_{xy}$ of each coil. At this time, a magnetic field map 720 formed by all shim channels may have a value similar to the inhomogeneous static magnetic field map 710 before shimming. Thus, the MRI apparatus 400 may obtain a relatively homogeneous static magnetic field map 730 compared to the inhomogeneous static magnetic field map 710 before shimming by offsetting the inhomogeneous static magnetic field map 710 before shimming with the magnetic field map 720 by each shim channel function. Specifically, an offset 731 of the static magnetic field map 730 after shimming may be obtained in a blurred density compared with the offsets 711 to 714 of the static magnetic field map 710 before shimming. That is, the offset 731 of a frequency relatively lower than the offsets 711 to 714 of the static magnetic field map 710 before shimming may be detected in the static magnetic field map 730 after shimming by the shim coil.

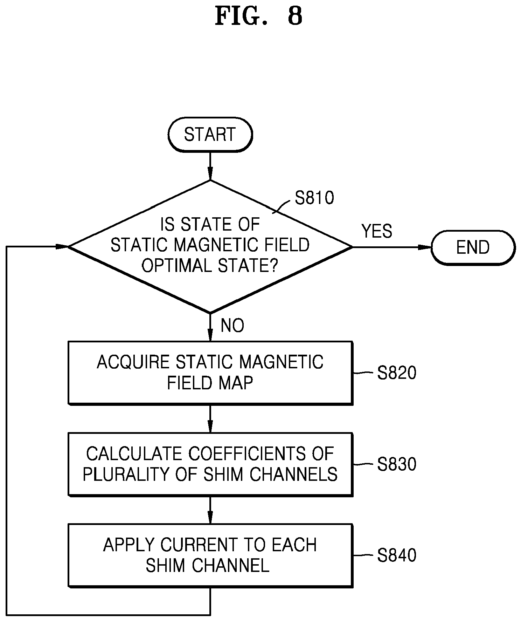

FIG. 8 is a flowchart illustrating a method performed by the MRI apparatus 400 of controlling repetitive high-order shimming, according to an embodiment.

The MRI apparatus 400 may determine whether a state of a static magnetic field is an optimal state for acquiring an MR image before shimming (S810). The optimal state of a magnetic field to acquire the MR image may mean that the magnetic field is homogeneous enough to minimize image distortion. When the MRI apparatus 400 determines that the state of the static magnetic field is the optimal state, the MRI apparatus 400 may end repetitive high-order shimming and control to operate an imaging protocol for acquiring the MR image. On the other hand, when the MRI apparatus 400 determines that the state of the static magnetic field is not the optimal state, the MRI apparatus 400 may control high-order shimming to be repeated again.

In an embodiment, the MRI apparatus 400 may use a FID signal as a reference for determining whether the state of the static magnetic field is the optimal state.

The FID signal may have a unique value for each tissue within an object according to magnitude of a main magnetic field. When the static magnetic field is in a homogeneous state, the FID signal in a specific tissue in the object may be detected with the same value as a unique FID signal value of the specific tissue. On the other hand, when the static magnetic field is in an inhomogeneous state, the FID signal detected in the specific tissue in the object may be detected with a value different from the unique FID signal value of the specific tissue due to a distortion of the static magnetic field. Therefore, when the FID signal obtained before shimming match the unique FID signal value of the specific tissue, the MRI apparatus 400 may determine that the state of the static magnetic field is the optimal state.

In an embodiment, the MRI apparatus 400 may use a FID signal of each of fat and water as a reference for determining whether the state of the static magnetic field is the optimal state. Specifically, a shimming stop control operation may be performed by the control unit 420.

The currently used MRI apparatus 400 may acquire an image using a magnetic moment of a $^1$H atomic nucleus. This is because a high density of $^1$H is present in a human body and the $^1$H atomic nucleus emits a very strong magnetic resonance signal. Therefore, the most MRI apparatuses 400 acquire image signals by applying a frequency adjacent to the Larmor frequency of $^1$H at a resonance frequency. Since water and fat include $^1$H in each molecule, water and fat may emit relatively large signals when acquiring magnetic resonance images. At this time, since a water molecule and a fat molecule have different molecular structures, the Larmor frequency is different. When the magnetic field state is homogeneous, the MRI apparatus 400 may acquire the FID signal of each of fat and water and perform 1D Fourier transform to detect one large peak (FID signal of water, hereinafter referred to as a first peak) and one other small peak (FID signal of fat, hereinafter referred to as a second peak) at different frequencies.

As described above, the MRI apparatus 400 may determine whether the state of the static magnetic field is the optimal state for acquiring an image based on the relatively strong FID signals of water and fat.

In an embodiment, the MRI apparatus 400 may determine whether the state of the static magnetic field is the optimal state using the FID signal of each of fat and water obtained at a 'specific region' of the object.

The FID signal of fat acquired by the MRI apparatus 400 may be weak compared to the FID signal of water. To detect a relatively large FID signal of fat in the object, the MRI apparatus 400 may use the FID signal of each of fat and water of a specific region (for example, thigh, abdomen, etc.) where a relatively large amount of fat is distributed.

In an embodiment, the MRI apparatus 400 may determine whether the state of the static magnetic field is the optimal state by using a FID signal frequency difference between fat and water. Specifically, the shimming stop control operation may be performed by the control unit 420.

The MRI apparatus 400 may determine that the state of the static magnetic field is the optimal state when an interval between the first peak and the second peak in a 1D Fourier transform graph of the FID signal corresponds to a reference value. Since a specific tissue has a unique FID signal, when the static magnetic field is homogeneous, the FID signal frequency difference between fat and water obtained by the MRI apparatus 400 may have a certain value.

The FID signal frequency difference between fat and water under 3 Tesla of a main magnetic field may be obtained as shown in Equation 1 above.

Therefore, the MRI apparatus 400 may control to stop repetitive high-order shimming when the interval between the first peak and the second peak is 434 Hz under 3 Tesla of the main magnetic field.

The MRI apparatus 400 may acquire a static magnetic field map through the RF coil 411 (S820).

The MRI apparatus 400 may calculate an offset based on the static magnetic field map and calculate a plurality of shim channel coefficients (S830).

In an embodiment, a shim coil may include a plurality of shim channels corresponding to a plurality of linear coils or high-order coils.

In an embodiment, the MRI apparatus 400 may form a shim channel function by each shim channel.

In an embodiment, the MRI apparatus 400 may calculate a coefficient of each shim channel function that may homogenize contrast of the static magnetic field map.

The MRI apparatus 400 may apply current corresponding to the coefficient of each shim channel function to each shim channel (S840). When the current is applied, a magnetic field may be formed by each shim channel function. At this time, the magnetic field formed by a sum of shim channel functions may be homogenized by offsetting an inhomogeneous magnetic field.

Only a method of calculating the coefficient of each shim channel based on the static magnetic field map and offsetting an inhomogeneous magnetic field is described as a method performed by the MRI apparatus 400 of performing shimming, but the shimming method is not limited thereto.

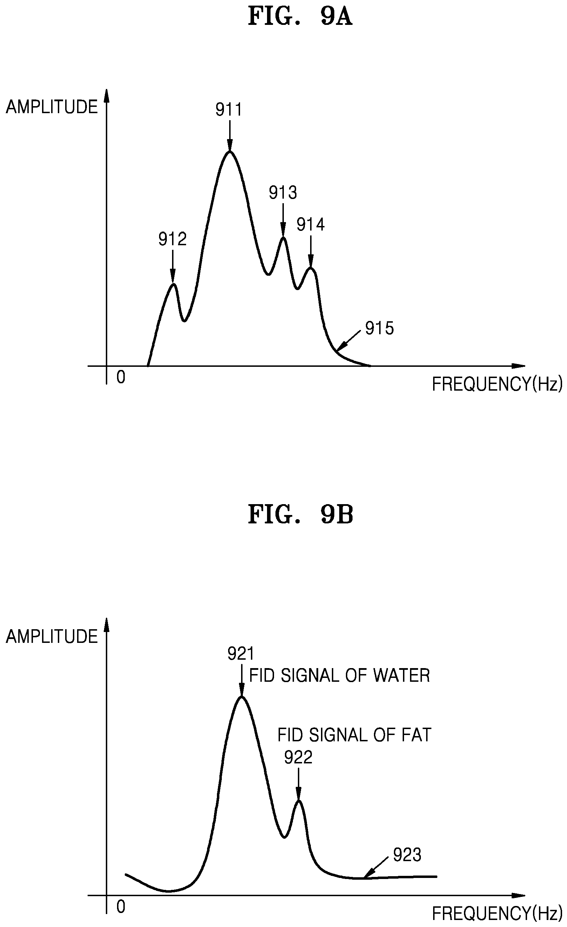

FIG. 9A is a graph schematically showing an FID signal obtained by the MRI apparatus 400 in an inhomogeneous state of a static magnetic field before completing shimming, according to an embodiment.

When a static magnetic field is inhomogeneous, a frequency value at each position of the bore 410 may vary, and thus distorted signal values other than FID signals of water and fat may also be detected (FIG. 9A). Specifically, a peak value 911 corresponding to the FID signal of water, and peak values 912, 913 and 914 of FID signals of other materials may be detected. Therefore, the FID signal of fat corresponding to a relatively small signal may not be clearly detected or may be difficult to distinguish from FID signals of other materials.

FIG. 9B is a graph schematically showing a FID signal obtained by the MRI apparatus 400 after homogenously completing shimming on a static magnetic field, according to an embodiment.

When the MRI apparatus 400 homogenously completes shimming on the static magnetic field, not only a peak value 921 of a FID signal of water but also a peak value 922 of a FID signal of fat corresponding to a relatively small signal may be detected.

The currently used MRI apparatus 400 may acquire an image using a magnetic resonance phenomenon of a $^1$H atomic nucleus. Accordingly, when the MRI apparatus 400 applies a frequency using the RF coil 411, a signal emitted from the $^1$H atomic nucleus existing in water and fat molecules may be detected.

Since other materials present in a human body are different from the $^1$H atomic nucleus in a Larmor frequency, the MRI apparatus 400 may not well detect a signal by other materials that do not include the $^1$H atomic nucleus. Accordingly, when the MRI apparatus 400 detects the FID signal after completing shimming, only two peaks corresponding to the FID signal of water and the FID signal of fat may be detected (921, 922).

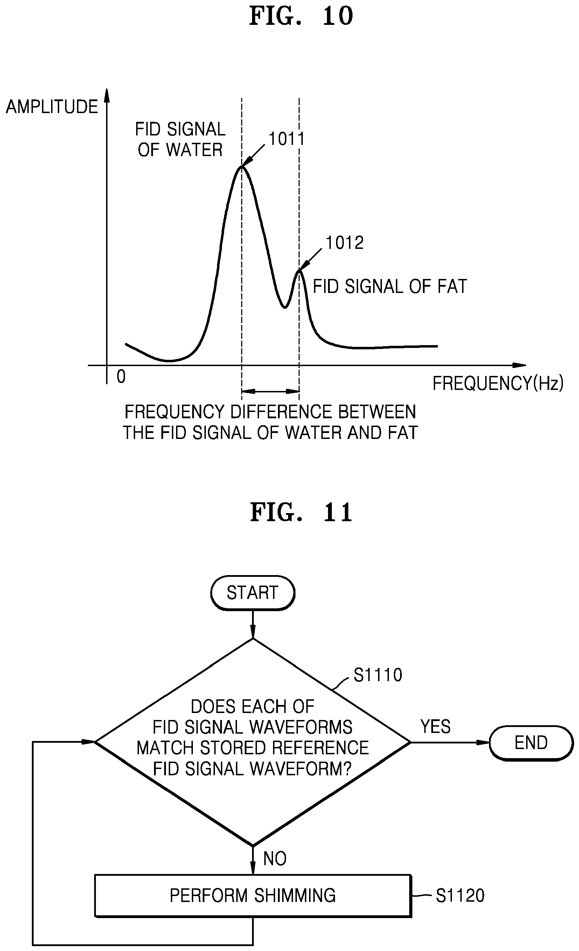

FIG. 10 is a graph showing a frequency difference between a FID signal of water and a FID signal of fat obtained by the MRI apparatus 400, according to an embodiment.

Referring to FIG. 10, when the MRI apparatus 400 completes shimming, FID signals having only two peaks 1011 and 1012 may be detected. At this time, the signal 1011 corresponding to a relatively large peak is the FID signal of water (hereinafter referred to as a first peak). The signal 1012 corresponding to a relatively small peak is the FID signal of fat (hereinafter referred to as a second peak).

In an embodiment, the MRI apparatus 400 may set a value of a frequency difference Δf between the first peak 1011 and the second peak 1012 as a control reference for repetitive high-order shimming. The frequencies of the first peak 1011 and the second peak 1012 are Larmor frequencies of water and fat, respectively. Under 3 Tesla of a main magnet field, a reference value of the frequency difference Δf between the first peak 1011 and the second peak 1012 may be

$$\triangle f = 42.58 MHz/Tesla * 3 Tesla * 3.4 * 10^{-6} \doteq 434 Hz.$$

When Δf corresponds to the reference value based on a characteristic that a Larmor frequency is unique for each material, the MRI apparatus 400 may control to stop repetitive high-order shimming. Specifically, a shimming stop control operation may be performed by the control unit 420.

In an embodiment, the MRI apparatus 400 may control whether to stop repetitive high-order shimming by using the FID signals 1011 and 1012 of water and fat on a 'specific part' of an object. Since an amount of fat tissue in a human body is not great, the MRI apparatus 400 may be difficult to detect the FID signal 1012 of fat clearly. Therefore, the FID 1012 signal of fat may be clearly detected by detecting a FID signal in the specific part (for example, abdomen, thigh, etc.) where a great amount of fat tissue is relatively distributed.

FIG. 11 is a flowchart illustrating a method performed by the MRI apparatus 400 of controlling repetitive high-order shimming according to whether each of a FID signal waveform of water and a FID signal waveform of fat matches a reference FID signal waveform, according to an embodiment.

According to an embodiment, the MRI apparatus 400 may determine whether each of the FID signal waveform of water and the FID signal waveform of fat matches the reference FID signal waveform before performing shimming (S1110). That is, the MRI apparatus 400 may determine whether each of the FID signal waveform of water and the FID signal waveform of fat matches the reference FID signal waveform, and when each of the FID signal waveform of water and the FID signal waveform of fat matches the reference FID signal waveform, end repetitive shimming to proceed with an imaging protocol for MR image acquisition.

Specifically, whether each of the FID signal waveform of water and the FID signal waveform of fat matches the reference FID signal waveform may be determined based on the number of peaks of each of the FID signal waveform of water and the FID signal waveform of fat, an interval between the peaks, heights of the peaks, and the like.

In an embodiment, when the FID signal waveform of water is referred to as a first peak and the local FID signal waveform is referred to as a second peak, the MRI apparatus 400 may control to stop repetitive high-order shimming when an interval between the first and second peaks in the FID signals acquired by the MRI apparatus 400 matches an interval between first and second peaks of the reference FID signal waveform. At this time, the 'interval between two peaks' may correspond to $\Delta f$. When shimming is completed, since the first peak and the second peak have values at a Larmor frequency of water and fat, respectively, the interval between the two peaks may correspond to $\Delta f$.

In an embodiment, the MRI apparatus 400 may control to stop repetitive high-order shimming when heights of remaining peaks except for the first and second peaks of the FID signals acquired by the MRI apparatus 400 match heights of remaining peaks except for the first and second peaks of the reference FID signal waveform, respectively. When the MRI apparatus 400 completes shimming, since only the FID signals of water and fat may be detected, remaining peak values except for the first and second peaks may be small or the remaining peaks may not be detected.

In an embodiment, the MRI apparatus 400 may control to stop repetitive high order shimming when the number of peaks of the FID signal received through the RF coil 411 matches the number of peaks of the reference FID signal.

The fact that the waveform of the FID signal acquired by the MRI apparatus 400 matches the waveform of the reference FID signal indicates that not only exact numerical values matches but also a difference between numerical values matches an error range that may be tolerated by one of ordinary skilled in the art.

When the MRI apparatus 400 determines that the FID signal waveform of each of water and fat does not match the reference FID signal waveform, the MRI apparatus 400 may proceed with shimming (S1120).

In an embodiment, the MRI apparatus 400 may include acquiring a static magnetic field map for shimming.

In an embodiment, the MRI apparatus 400 may calculate a shim channel coefficient based on the static magnetic field map.

In an embodiment, the MRI apparatus 400 may apply current corresponding to the shim channel coefficient to each shim coil to form a magnetic field.

In an embodiment, the MRI apparatus 400 may homogenize a static magnetic field with the magnetic field formed by the shim coil.

In an embodiment, the MRI apparatus 400 may perform shimming by other methods as well as shimming by the shim coil.

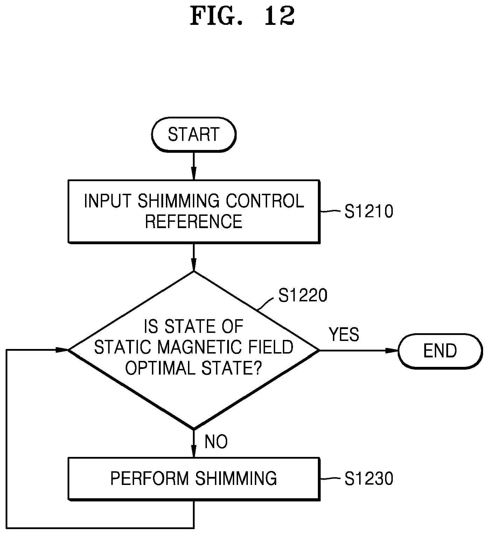

FIG. 12 is a flowchart illustrating a method performed by the MRI apparatus 400 of receiving a shimming control reference from a user and controlling repetitive high-order shimming, according to an embodiment.

The MRI apparatus 400 may receive a stop control reference of repetitive high-order shimming from the user (S1210). Specifically, the MRI apparatus 400 may receive the stop control reference of repetitive high-order shimming through the user input unit 430.

In an embodiment, the MRI apparatus 400 may display a user input window for receiving the shimming stop reference from the user.

In an embodiment, the user input window of the MRI apparatus 400 may include an FID signal as the repetitive shimming control reference.

In an embodiment, the user input window of the MRI apparatus 400 may include the FID signal of each of the water and the fat as a repetitive shimming control reference.

In an embodiment, the user input window of the MRI apparatus 400 may include the FID signal for each of water and fat in the 'specific part' of the object as the repetitive shimming control reference.

In an embodiment, the user input window of the MRI apparatus 400 may include a frequency difference of the FID signals of water and fat as the repetitive shimming control reference.

In an embodiment, the user input window of the MRI apparatus 400 may include whether a FID signal waveform of each of fat and water matches a reference FID signal waveform as the repetitive shimming control reference.

In an embodiment, the user input window of the MRI apparatus 400 may include that no FID signal other than the FID signal of each of fat and water is detected as the repetitive shimming control reference.

The MRI apparatus 400 may determine whether a state of a static magnetic field before shimming is an optimal state for acquiring an MR image (S1220).

The state of the static magnetic field is the optimal state for acquiring the MR image may include that the static magnetic field is in a homogeneous state enough to minimize image distortion. When the MRI apparatus 400 determines that the state of the static magnetic field is the optimal state, the MRI apparatus 400 may end repetitive high-order shimming and control an imaging protocol for acquiring the MR image to operate. On the other hand, when the MRI apparatus 400 determines that the state of the static magnetic field is inhomogeneous, the MRI apparatus 400 may control high-order shimming to proceed.

In an embodiment, the MRI apparatus 400 may use the FID signal as a reference for determining whether the state of the static magnetic field is the optimal state. Specifically, a shimming stop control operation may be performed by the control unit 420.

The FID signal may have a unique value according to magnitude of the static magnetic field. When the static magnetic field is in the homogeneous state, a FID signal in a specific tissue of the object may be detected with the same value as the unique FID signal value of the specific tissue. On the other hand, when the static magnetic field is inhomogeneous, the FID signal detected in the specific tissue of the object may be detected with a different value from the unique FID signal value of the specific tissue due to a distortion of the static magnetic field. Therefore, the MRI apparatus 400 may determine whether the state of the static magnetic field is the optimal state based on whether the FID signal matches the unique FID signal value of the specific tissue.

In an embodiment, the MRI apparatus 400 may use the FID signal of each of fat and water as the reference for determining whether the state of the static magnetic field is the optimal state. Specifically, the shimming stop control operation may be performed by the control unit 420.

The currently used MRI apparatus 400 may acquire an image using a magnetic moment of a $^1$H atomic nucleus. This is because a high density of $^1$H is present in a human body and the $^1$H atomic nucleus emits a very strong magnetic resonance signal. Therefore, the most MRI apparatuses 400 acquire image signals by applying a frequency adjacent to the Larmor frequency of $^1$H at a resonance frequency.

Since water and fat include $^1$H in each molecule, water and fat may emit relatively large signals when acquiring magnetic resonance images. At this time, since a water molecule and a fat molecule have different molecular structures, the Larmor frequency is different. When the magnetic field state is homogeneous, the MRI apparatus 400 may acquire the FID signal of each of fat and water and perform 1D Fourier transform to detect one large peak (FID signal of water, hereinafter referred to as a first peak) and one other small peak (FID signal of fat, hereinafter referred to as a second peak) at different frequencies.

In an embodiment, the MRI apparatus 400 may use the FID signal of each of fat and water in the 'specific part' of the object as the reference for determining whether the state of the static magnetic field is the optimal state.

The FID signal of fat acquired by the MRI apparatus 400 may not be clear since the FID signal of fat is less than the FID signal of water. Therefore, to detect a relatively large fat FID signal, the MRI apparatus 400 may use the FID signal of each fat and water in a specific part (for example, thigh, abdomen, etc.) where a large amount of fat tissue is distributed.

In an embodiment, the MRI apparatus 400 may use the FID signal frequency difference between fat and water as the reference for determining whether the state of the static magnetic field is the optimal state. Specifically, the shimming stop control operation may be performed by the control unit 420.

When an interval between the first peak and the second peak in the 1D Fourier transform graph of the detected FID signal corresponds to a reference value Δf, the MRI apparatus 400 may determine that the state of the static magnetic is the optimal state. Since the specific tissue has a unique FID signal, when the static magnetic field is homogeneous, the FID signal frequency difference between fat and water acquired by the MRI apparatus 400 may also have a certain value.

The FID signal frequency difference between fat and water under 3 Tesla of a main magnetic field may be obtained as follows.

$$\triangle f = 42.58 MHz/Tesla * 3Tesla * 3.4 * 10^{-6} \fallingdotseq 434 Hz$$

The MRI apparatus 400 may control to stop repetitive high-order shimming when the interval between the first peak and the second peak is 434 Hz in the case where the main magnetic field is 3 Tesla.

When the MRI apparatus 400 determines that the state of the static magnetic field is inhomogeneous, the MRI apparatus 400 may perform shimming (S1230).

In an embodiment, the MRI apparatus 400 may acquire a static magnetic field map through the RF coil 411.

In an embodiment, the MRI apparatus 400 may calculate an offset based on the static magnetic field map and calculate coefficients of a plurality of shim channels. That is, the MRI apparatus 400 may calculate the coefficient of each shim channel to form shim channel functions that may offset an inhomogeneous static magnetic field.

In an embodiment, the MRI apparatus 400 may apply current corresponding to the coefficient of each shim channel to form a magnetic field. At this time, the magnetic field formed by a sum of the shim channel functions may be homogenized by offsetting the inhomogeneous magnetic field.

Although it is described above that the shimming method performed by the MRI apparatus 400 only includes offsetting the inhomogeneous magnetic field by calculating the coefficient of each shim channel based on the static magnetic field map, the disclosure is not limited thereto and the shimming method may include other methods.

As described above, an MRI apparatus and a shimming method of the MRI apparatus according to an embodiment determine a state of a magnetic field based on a FID signal and control whether to stop shimming based on determination, thereby reducing the number of times of shimming and improving homogeneity of the magnetic field.

Specifically, the MRI apparatus and the shimming method thereof according to an embodiment may control whether to stop shimming based on at least one of offsets of a plurality of shim channels and a FID signal of each of fat and water received from a RF coil, thereby more accurately controlling to stop shimming.

The embodiments of the present disclosure may be written as computer programs and may be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs or DVDs), etc.

While the present disclosure has been particularly shown and described with reference to example embodiments thereof, it will be understood by those of ordinary skill in the art that various changes in form and details may be made therein without departing from the spirit and scope of the present disclosure as defined by the following claims.

**Claims**

1. A shimming method performed by a magnetic resonance imaging (MRI) apparatus, the shimming method comprising:

   applying a magnetic field and a high frequency to an object through a bore of the MRI apparatus comprising at least one magnet and an RF coil and obtaining a static magnetic field map corresponding to the magnetic field formed in the bore;
   performing shimming using a plurality of shim channels based on the static magnetic field map;
   receiving a free induction decay (FID) signal from the RF coil; and
   determining a state of the magnetic field based on the FID signal and controlling whether to stop shimming based on the determined state.

2. The shimming method of claim 1, wherein the performing of the shimming comprises:

   calculating offsets of the plurality of shim channels based on the static magnetic field map; and
   applying a current to the plurality of shim channels based on the calculated offsets.

3. The shimming method of claim 1, wherein the controlling whether to stop shimming comprises controlling whether to stop shimming by using a FID signal for each of fat and water received from the RF coil.

4. The shimming method of claim 3, wherein the FID signal for each of fat and water is an FID signal with respect to a specific part of the object.

5. The shimming method of claim 3, wherein the controlling whether to stop shimming comprises controlling to stop shimming when a difference between a frequency value of the FID signal for fat and a frequency value of the FID signal for water matches a reference value.

6. The shimming method of claim 3, wherein the controlling whether to stop shimming comprises controlling to stop shimming when a waveform of the FID signal for each of fat and water matches a waveform of a reference FID signal.

7. The shimming method of claim 3, wherein the controlling whether to stop shimming comprises controlling to stop shimming when an FID signal other than the FID signal for each of fat and water is not detected.

8. The shimming method of claim 1, wherein the controlling whether to stop shimming comprises receiving a reference for controlling a user to stop shimming.

9. A magnetic resonance imaging (MRI) apparatus comprising:

   a bore comprising at least one magnet and an RF coil and configured to apply a magnetic field and a high frequency to an object; and
   a control unit configured to obtain a static magnetic field map corresponding to the magnetic field formed in the bore based on a high frequency signal received from the RF coil, to perform shimming using a plurality of shim channels based on the static magnetic field map, to determine a state of the magnetic field based on the FID signal, and to control whether to stop shimming based on the determined state.

10. The MRI apparatus of claim 9, wherein the control unit is configured to calculate offsets of the plurality of shim channels based on the static magnetic field map and apply a current to the plurality of shim channels based on the calculated offsets.

11. The MRI apparatus of claim 9, wherein the control unit is configured to control whether to stop shimming by using a FID signal for each of fat and water received from the RF coil.

12. The MRI apparatus of claim 11, wherein the FID signal for each of fat and water is an FID signal with respect to a specific part of the object.

13. The MRI apparatus of claim 11, wherein the control unit is configured to control to stop shimming when a difference between a frequency value of the FID signal for fat and a frequency value of the FID signal of water matches a reference value.

14. The MRI apparatus of claim 11, wherein the control unit is configured to control to stop shimming when a waveform of the FID signal for each of fat and water matches a waveform of a reference FID signal.

15. The MRI apparatus of claim 11, wherein the control unit is configured to control to stop shimming when an FID signal other than the FID signal for each of fat and water is not detected.

16. The MRI apparatus of claim 9, wherein the control unit is configured to receive a reference for controlling a user to stop shimming.

# FIG. 1

**MONITORING UNIT 40**
- SYSTEM MONITORING UNIT 42
- OBJECT MONITORING UNIT 44
- TABLE CONTROLLER 46
- DISPLAY CONTROLLER 48

**OPERATING UNIT 60**
- INPUT UNIT 66
- OUTPUT UNIT 64
- IMAGE PROCESSOR 62

**SYSTEM CONTROL UNIT 50**
- GANTRY CONTROLLER 58
- SEQUENCE CONTROLLER 52
- GRADIENT MAGNETIC FIELD CONTROLLER 54
- RF CONTROLLER 56

**SIGNAL TRANSCEIVER 30**
- GRADIENT AMPLIFIER 32
- TRANSMISSION AND RECEPTION SWITCH 34
- RF TRANSMITTER 36
- RF RECEIVER 38

20 22 24 26 26 29 10 28

# FIG. 2

# FIG. 3

300

310

BORE

311

RF COIL

312

MAIN
MAGNET

320

CONTROL UNIT

# FIG. 4

# FIG. 5

START

ACQUIRE STATIC MAGNETIC FIELD MAP — S510

PERFORM SHIMMING USING PLURALITY OF SHIM CHANNELS — S520

RECEIVE FID SIGNAL — S530

DETERMINE STATE OF MAGNETIC FIELD BASED ON FID SIGNAL AND CONTROL WHETHER TO STOP SHIMMING — S540

END

# FIG. 6A

$B_i(Z)$

FIELD BEFORE SHIMMING

410

611

REGION OF INTEREST

Z

# FIG. 6B

$C_jS_j(Z)$

SHIM CHANNEL FUNCTIONS

Z4
Z3
410
Z
612
Z1
Σ
Z2

# FIG. 6C

$B_{residual} (Z)$

FIELD AFTER SHIMMING

410

613

REGION OF INTEREST

Z

# FIG. 7

INHOMOGENEOUS STATIC MAGNETIC
FIELD MAP BEFORE SHIMMING

HOMOGENEOUS STATIC MAGNETIC
FIELD MAP AFTER SHIMMING

711
712
713
714

710

731

730

720

SUBTRACTION

CALCULATE COIL COEFFICIENT
• LINEAR COILS : X, Y, Z
• HIGH-ORDER COILS : $Z^2$, ZX, ZY, $X^2-Y^2$, XY

$$Z^2 \times C_{z^2} + ZX \times C_{zx} + \cdots + (X^2-Y^2) \times C_x^2 - Y^2 + XY \times C_{XY} =$$

# FIG. 8

START

S810

IS STATE OF
STATIC MAGNETIC FIELD
OPTIMAL STATE?

YES

END

NO

ACQUIRE STATIC MAGNETIC
FIELD MAP

S820

CALCULATE COEFFICIENTS OF
PLURALITY OF SHIM CHANNELS

S830

APPLY CURRENT TO EACH
SHIM CHANNEL

S840

# FIG. 9A

# FIG. 9B

# FIG. 10

AMPLITUDE

FID SIGNAL
OF WATER

1011

1012

FID SIGNAL OF FAT

0

FREQUENCY(Hz)

FREQUENCY DIFFERENCE BETWEEN
THE FID SIGNAL OF WATER AND FAT

# FIG. 11

START

S1110

DOES EACH OF
FID SIGNAL WAVEFORMS
MATCH STORED REFERENCE
FID SIGNAL WAVEFORM?

YES

END

NO

PERFORM SHIMMING — S1120

# FIG. 12

```
        ┌─────────┐
        │  START  │
        └────┬────┘
             ▼
   ┌──────────────────────┐
   │ INPUT SHIMMING CONTROL│── S1210
   │      REFERENCE        │
   └──────────┬───────────┘
              ▼
          ╱────────╲           S1220
         ╱ IS STATE  ╲      YES    ┌─────┐
        ╱ OF STATIC    ╲─────────▶│ END │
        ╲ MAGNETIC FIELD╱          └─────┘
         ╲OPTIMAL STATE?╱
          ╲────────╱
              │ NO
              ▼
   ┌──────────────────────┐
   │   PERFORM SHIMMING    │── S1230
   └──────────────────────┘
```

## INTERNATIONAL SEARCH REPORT

| International application No. |
|---|
| **PCT/KR2016/010827** |

| A. CLASSIFICATION OF SUBJECT MATTER |
|---|
| *A61B 5/055(2006.01)i, A61B 5/00(2006.01)i, G01R 33/32(2006.01)i* |
| According to International Patent Classification (IPC) or to both national classification and IPC |

| B. FIELDS SEARCHED |
|---|
| Minimum documentation searched (classification system followed by classification symbols) |
| A61B 5/055; G01V 3/00; G01R 33/20; G01R 33/46; G01R 33/3875; G01R 33/48; G01R 33/54; A61B 5/00; G01R 33/32 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: MRI, shimming, control, FID, water, fat |

| C. DOCUMENTS CONSIDERED TO BE RELEVANT | | |
|---|---|---|
| Category* | Citation of document, with indication, where appropriate, of the relevant passages | Relevant to claim No. |
| Y | US 6529002 B1 (KIM et al.) 04 March 2003<br>See abstract, column 2, line 22-column 5, line 16 and claim 1. | 9-16 |
| Y | US 2015-0077107 A1 (NATIONAL RESEARCH COUNCIL OF CANADA)<br>19 March 2015<br>See abstract, paragraph [8] and claims 1-7. | 9-16 |
| Y | US 2013-0088226 A1 (MIYAZAKI) 11 April 2013<br>See abstract, paragraphs [20], [21] and claim 6. | 11-16 |
| A | US 4899109 A (TROPP et al.) 06 February 1990<br>See column 9, lines 3-37 and claims 1-9. | 9-16 |
| A | US 2010-0102819 A1 (ZAITSEV et al.) 29 April 2010<br>See paragraphs [20], [21] and claims 1, 2. | 9-16 |

| ☐ Further documents are listed in the continuation of Box C. | ☒ See patent family annex. |
|---|---|

| * Special categories of cited documents: | "T" | later document published after the international filing date or priority date and not in conflict with the application but cited to understand the principle or theory underlying the invention |
|---|---|---|
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| 05 JANUARY 2017 (05.01.2017) | **06 JANUARY 2017 (06.01.2017)** |

| Name and mailing address of the ISA/KR | Authorized officer |
|---|---|
| Korean Intellectual Property Office<br>Government Complex-Daejeon, 189 Seonsa-ro, Daejeon 302-701,<br>Republic of Korea | |
| Facsimile No. +82-42-481-8578 | Telephone No. |

Form PCT/ISA/210 (second sheet) (January 2015)

## INTERNATIONAL SEARCH REPORT

| International application No. |
| --- |
| **PCT/KR2016/010827** |

**Box No. II      Observations where certain claims were found unsearchable (Continuation of item 2 of first sheet)**

This international search report has not been established in respect of certain claims under Article 17(2)(a) for the following reasons:

1. ☒  Claims Nos.: **1-8**
   because they relate to subject matter not required to be searched by this Authority, namely:

   Claims 1 to 8 pertain to a diagnostic method, and thus pertain to subject matter on which the International Searching Authority is not required to carry out an international search under the provisions of PCT Article 17(2)(a)(i) and PCT Rule 39.1(iv).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☒  No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

INTERNATIONAL SEARCH REPORT
Information on patent family members

International application No.

**PCT/KR2016/010827**

| Patent document cited in search report | Publication date | Patent family member | Publication date |
|---|---|---|---|
| US 6529002 B1 | 04/03/2003 | NONE | |
| US 2015-0077107 A1 | 19/03/2015 | WO 2013-155606 A1 | 24/10/2013 |
| US 2013-0088226 A1 | 11/04/2013 | JP 2013-081780 A | 09/05/2013 |
| | | JP 5981293 B2 | 31/08/2016 |
| | | US 8749236 B2 | 10/06/2014 |
| US 4899109 A | 06/02/1990 | NONE | |
| US 2010-0102819 A1 | 29/04/2010 | US 8018230 B2 | 13/09/2011 |

Form PCT/ISA/210 (patent family annex) (January 2015)